# EUROPÄISCHE PATENTSCHRIFT

(11) **EP 2 748 353 B1**
(45) Veröffentlichungstag und Bekanntmachung des Hinweises auf die Patenterteilung: **26.04.2017**
(21) Anmeldenummer: 12745879.2
(22) Anmeldetag: 14.08.2012
(51) Int. Cl.: C25B 3/04, C07D 307/33

(54) **VERFAHREN ZUR ELEKTROCHEMISCHEN DARSTELLUNG VON GAMMA-HYDROXYCARBONSÄUREESTERN UND GAMMA-LACTONEN**
METHOD FOR THE ELECTROCHEMICAL PRODUCTION OF GAMMA-HYDROXYCARBOXYLIC ESTERS AND GAMMA-LACTONES
PROCÉDÉ DE PRÉPARATION ÉLECTROCHIMIQUE D'ESTERS D'ACIDE GAMMA-HYDROXYCARBOXYLIQUE ET DE GAMMA-LACTONES

(30) Priorität: 24.08.2011 EP 11178688
(43) Veröffentlichungstag der Anmeldung: 02.07.2014
(73) Patentinhaber: BASF SE, 67056 Ludwigshafen am Rhein (DE)
(72) Erfinder: MALKOWSKY, Itamar Michael, 67346 Speyer (DE); STECKER, Florian, 68167 Mannheim (DE); LUTTER, Simone, 67069 Ludwigshafen (DE); ABILLARD, Olivier, 68159 Mannheim (DE); PELZER, Ralf, 37699 Fürstenberg (DE)
(86) Internationale Anmeldenummer: PCT/EP2012/065858
(87) Internationale Veröffentlichungsnummer: WO 2013/026737

(56) Entgegenhaltungen:
- EP-A1- 0 635 587
- GB-A- 1 447 772
- JP-A- S5 813 572
- JP-A- 57 108 274
- US-A- 4 011 177
- US-A- 4 414 079
- US-B1- 6 437 150
- Tatsuo Shono ET AL: "Electroreductive hydrocoupling of activated olefins with ketones or aldehydes in the presence of trimethylchlorosilane", Tetrahedron Letters, 1980, Seiten 5029-5032, XP055016860, Great Britain Gefunden im Internet: URL:http://pdn.sciencedirect.com/science?_ ob=MiamiImageURL&_cid=271373&_user=987766& _pii=S0040403900711245&_check=y&_origin=br owse&_zone=rslt_list_item&_coverDate=1980- 12-31&wchp=dGLbVlB-zSkzV&md5=20c9767a517c9 28ebf9f3a08b440ba65/1-s2.0-S00404039007112 45-main.pdf [gefunden am 2012-01-18] in der Anmeldung erwähnt
- ZIEGLER, F.E. ET AL.: "Applications of the 3-methyl-gamma-butyrolactone strategy to the synthesis of polypropionates: the Prelog-Djerassi lactonic ester, ent-invictolide, and the C19-C27 fragment of rifamycin S", JOURNAL OF THE AMERICAN CHEMICAL SOCIETY, Bd. 110, Nr. 16, 1988, Seiten 5442-5452, XP55075201, ISSN: 0002-7863, DOI: 10.1021/ja00224a032
- ZIEGLER, F.E. ET AL.: "R-3-methyl-gamma-butyrolactone as a template for the synthesis of (+)-invictolide", TETRAHEDRON LETTERS, Bd. 27, Nr. 11, 1986, Seiten 1229-1232, XP55075203, ISSN: 0040-4039, DOI: 10.1016/S0040-4039(00)84224-0
- NIKISCHIN, G.I. ET AL.: "Free-Radical Addition of Acohols to Acrylic Acid and its Methyl Ester. Synthesis of gamma-Lactones", DOKLADY. CHEMISTRY, MAIK NAUKA/INTERNPERIODICA PUBLISHING, MOSCOW, RU, Bd. 136, 1961, Seiten 55-58, XP009171883, ISSN: 0012-5008
- BODANSZKY, M. ET AL.: "THE STRUCTURE OF FATTY ACIDS FROM THE ANTIBIOTIC AMPHOMYCIN", JOURNAL OF ANTIBIOTICS, JAPAN ANTIBIOTICS RESEARCH ASSOCIATION, TOKYO, JP, Bd. 22, Nr. 9, 1969, Seiten 399-408, XP009171902, ISSN: 0021-8820, DOI: 10.7164/ANTIBIOTICS.22.399 [gefunden am 2006-04-12]
- SINHA, A.K. ET AL.: "Microwave-Assisted Mild Conversion of Natural Dihydrotagetone into 5-Isobutyl-3-methyl-4,5-dihydro-2(3H)-fura none, an Analogue of Whisky Lactone", AUSTRALIAN JOURNAL OF CHEMISTRY, Bd. 60, Nr. 2, 2007, Seiten 124-127, XP009171901, ISSN: 0004-9425, DOI: 10.1071/CH06380 [gefunden am 2007-02-13]
- NAZAROV, I.N. ET AL.: "Acetylene derivatives Communication 191. Preparation of acids from tertiary acetylenic alcohols", BULLETIN OF THE ACADEMY OF SCIENCES OF THE USSR, DIVISION OF CHEMICAL SCIENCES, SPRINGER NEW YORK LLC, US, Bd. 7, Nr. 11, 1958, Seiten 1306-1312, XP009171905, ISSN: 0568-5230, DOI: 10.1007/BF00913518
- MOROE, T. ET AL.: "Synthesis of Isocitronellol (Synthetic Perfume). I.", YAKUGAKU ZASSHI, PHARMACEUTICAL SOCIETY OF JAPAN, JP, [Online] Bd. 72, Nr. 9, 1952, Seiten 1172-1174, XP009171904, ISSN: 0031-6903 Gefunden im Internet: URL:JST.Journalarchive/yakushi1947/72.9_11 72> [gefunden am 2010-02-19]
- BROWN, H.C. ET AL.: "Chiral Synthesis via Organoboranes. 39. A Facile Synthesis of gamma-Substituted-gamma-butyrolactones in Exceptionally High Enantiomeric Purity", THE JOURNAL OF ORGANIC CHEMISTRY, Bd. 59, Nr. 2, 1994, Seiten 365-369, XP55075287, ISSN: 0022-3263, DOI: 10.1021/jo00081a014
- GASSMAN, P.G. ET AL.: "Photoinduced lactonization. A useful but mechanistically complex single electron transfer process", JOURNAL OF THE AMERICAN CHEMICAL SOCIETY, Bd. 109, Nr. 24, 1987, Seiten 7547-7548, XP55075292, ISSN: 0002-7863, DOI: 10.1021/ja00258a059
- CRONYN, M.W.: "Sulfones. I. Methods for the Preparation of Certain Alkanes, Alkenes, Acids and Lactones with Bis-(ethylsulfonyl)-methane", JOURNAL OF THE AMERICAN CHEMICAL SOCIETY, Bd. 74, Nr. 5, 1952, Seiten 1225-1230, XP55075322, ISSN: 0002-7863, DOI: 10.1021/ja01125a026
- JHA, V. ET AL.: "Enantioselective Synthesis of syn/anti -1,3-Amino Alcohols via Proline-Catalyzed Sequential alpha-Aminoxylation/alpha-Amination and Horner-Wadsworth-Emmons Olefination of Aldehydes", ORGANIC LETTERS, Bd. 12, Nr. 12, 2010, Seiten 2762-2765, XP55075333, ISSN: 1523-7060, DOI: 10.1021/ol100856u
- STURM, T.-J. ET AL.: "Diastereoselective reactions of ester enolates with epoxides", THE JOURNAL OF ORGANIC CHEMISTRY, Bd. 54, Nr. 9, 1989, Seiten 2039-2040, XP55075331, ISSN: 0022-3263, DOI: 10.1021/jo00270a007
- NAGANO, H. ET AL.: "Chelation-controlled highly diastereoselective catalytic hydrogenation of gamma-hydroxy-alpha-methylenecarboxylic acid esters", TETRAHEDRON LETTERS, Bd. 45, Nr. 15, 2004, Seiten 3035-3037, XP004496740, ISSN: 0040-4039, DOI: 10.1016/J.TETLET.2004.02.111
- NOLTES, A.W. ET AL.: "The Condensation of Ketones and the Ethyl Hemiacetal of Ethyl Glyoxylate", RECUEIL DES TRAVAUX CHIMIQUES DES PAYS-BAS, ELSEVIER SCIENCE PUBLISHERS, AMSTERDAM, NL, Bd. 80, 1961, Seiten 1334-1347, XP002150906, ISSN: 0165-0513

## Beschreibung

Die Erfindung betrifft Verfahren zur elektrochemischen Darstellung von γ-Hydroxycarbonsäureestern und γ-Lactonen durch reduktive Kreuzkopplung von α,β-ungesättigten Estern mit Carbonylverbindungen in einer ungeteilten Elektrolysezelle, wobei eine Kathode aus Blei, Bleilegierungen, Kadmium, Kadmiumlegierungen, Quecksilber, Stahl, Glaskohlenstoff oder Bordotierten Diamanten und ein basischer wässriger Elektrolyt mit einem Leitsalz, ausgewählt aus bisquartären und multiquartären Ammonium- und Phosphoniumsalzen, eingesetzt werden.

Die Anmeldung beschreibt weiter die γ-Butyrolacton-Derivate der Formel I 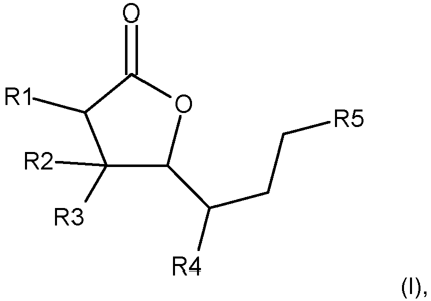 die nach dem erfindungsgemäßen Verfahren herstellbar sind, sowie deren Verwendung als Aromastoffe.

Die Anmeldung beschreibt auch die γ-Hydroxycarbonsäuren bzw. γ-Hydroxycarbonsäureester der Formel VII 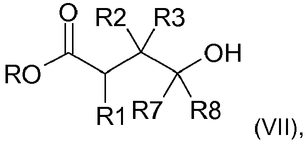 die ebenfalls nach dem erfindungsgemäßen Verfahren herstellbar sind.

Das technisch wichtigste γ-Lacton ist γ-Butyrolacton. Industriell wird es entweder durch Dehydrocyclisierung von 1,4-Butandiol in der Gasphase oder mittels Hydrierung von Maleinsäureanhydrid dargestellt. Eine weitere klassische Methode zur Darstellung von γ-Lactonen ist die alkalische Hydrolyse von γ-Halogencarbonsäuren.

Die zuvor beschriebenen Methoden gehen stets von einem bestehenden disubstituierten C₄-Gerüst aus, so dass Substitutionsmuster am Ring nicht konvergent realisiert werden können. Daneben sind aber auch Methoden bekannt, in denen der spätere Lactonring erst durch eine *C,C*-Bindungsknüpfung aufgebaut wird. Hierzu zählen bspw. die oxidative Verknüpfung von Essigsäure mit Olefinen (C₂ + C₂) oder die *tert*-Butylhydroperoxid-vermittelte Cyclisierung von Acrylsäure mit Alkoholen (C₃ + C₁). In diesen Fällen kann die Substitution am Ring durch geschickten Einsatz der entsprechenden Edukte in der Cyclisierung gesteuert werden.

Zu dieser Art der Lactonsynthese zählt auch die reduktive Verknüpfung (Dihydrodimerisierung) von Acrylsäureester und Carbonylverbindungen dem folgenden Reaktionsschema:

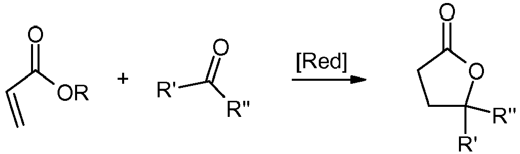

Die reduktive Kupplung von Acrylsäurederivaten mit Carbonylverbindungen kann durch Reduktionsmittel wie Magnesium oder Samarium(II)iodid vermittelt werden. Ferner sind elektrochemische Methoden beschrieben, welche den stöchiometrischen Einsatz eines chemischen Reduktionsmittels vermeiden. Grundlegende Arbeiten auf diesem Gebiet erfolgten in einer geteilten elektrochemischen Zelle an einer Quecksilber-Poolkathode in einem schwefelsauren Elektrolvten bei kathodischen Stromdichten von bis zu 2.8 A/dm². Im Rahmen dieser Arbeiten wurde die reduktive Kupplung von Acrylnitril mit Aceton betrachtet, was also noch nicht zu den Lactonen führt.

Hiervon ausgehend haben Shono et al. (Tetrahedron Lett. 1980, 21, 5029-5032) die reduktive Kupplung von α,β-ungesättigten Estern mit Aldehyden oder Ketonen in einer geteilten elektrochemischen Zelle beschrieben. Der eingesetzte Elektrolyt basierte auf N,N-Dimethylformamid (DMF) mit N,N,N,N-Tetraethylammoniumtoluolsulfonat (Et₄NOTs) als Leitsalz. Zur Aktivierung der Carbonylkomponente wurden ferner stöchiometrische Mengen eines Chlorsilans (Trimethylsilylchlorid, TMSCI) zugesetzt. Die Elektrolyse erfolgte bei einer Stromdichte von 0,4 A/dm², welche von technisch relevanten Stromdichten > 1 A/dm² weit entfernt ist. Nobuya et al. (JP 57108274 A) haben einen weiteren Schritt zur technischen Realisierung unternommen, indem ein Wasser-basierter Elektrolyt verwendet wurde. Die Darstellung der Lactone erfolgte in einer geteilten Elektrolysezelle bei Stromdichten von 10 A/dm². Hierbei wurden ein saurer Anolyt (z.B. 10 %ige H₂SO₄) und ein KH₂PO₄-gepufferter Katholyt verwendet. Bei geteilten Zellen sind die beiden Elektrodenräume durch eine Membran getrennt. Ungeteilte Zellen sind günstiger und technisch leichter zu realisieren. Insbesondere bei organischen Verfahren ist von einer schnellen Alterung der Membran auszugehen, und damit von unbefriedigenden Standzeiten.

US 4,414,079 beschreibt die Umsetzung von α,β-ungesättigten Estern mit Aldehyden auch in ungeteilten Zellen unter Einsatz von beispielsweise tetra-n-Butylammoniumsulfat als Leitsalz. In einem weiteren Ansatz hat Bürger (Katrin Bürger, Dissertation 2003, Universität Münster) die Umsetzung von α,β-ungesättigten Estern mit Aldehyden oder Ketonen in einer ungeteilten Zelle realisiert. Als Elektrolyte wurden binäre Gemische von Alkoholen (z.B. Methanol oder Ethanol) mit Wasser oder Dioxan sowie hohe Konzentrationen an Leitsalzen (z.B. Tetrabutylammoniumtetrafluoroborat, Bu₄NBF₄) eingesetzt. Interessanterweise wurden in dem beschriebenen System Graphitelektroden eingesetzt, die aufgrund ihrer vergleichsweise hohen Wasserstoffüberspannung als Alternative für Blei-, Quecksilber- und Cadmium-Elektroden dienen könnten. Die nach diesem Verfahren erhältlichen Ausbeuten an Lacton sind jedoch unbefriedigend, da sich in großem Ausmaß die entsprechenden *homo*-Kupplungsprodukte sowie die reduzierte Carbonylkomponente (also der entsprechende Alkohol) als Nebenprodukte bilden, obwohl der *homo-*Kopplung des α,β-ungesättigten Estern durch einen hohen Überschuss an Carbonylverbindung entgegengewirkt wird. Darüber hinaus beruht dieses Verfahren auf dem Einsatz von Elektrolyten auf Basis binärer organischer Lösemittel (Alkohol mit Wasser oder Alkohol mit Dioxan), womit eine aufwendige Abtrennung des Produkts von dem Lösemittel nach der Elektrolyse erforderlich ist. Der Einsatz von alkholhaltigen Lösemitteln ist auch deshalb von Nachteil, weil der Alkohol unter der Elektrolyse oxidiert wird (zum Aldehyd und weiter). Dadurch geht treures Lösemittel verloren und der entstandene Aldehyd muss aufwendig abgetrennt werden.

Als der Erfindung zugrunde liegende Aufgabe kann daher erachtet werden, die Bereitstellung eines Verfahrens zur elektrochemischen Darstellung von γ-Lactonen und γ-Hydroxycarbonsäureestern durch Kreuzkupplung von α,β-ungesättigten Estern mit Carbonylverbindungen bei dem die Nachteile des Standes der Technik, insbesondere der Einsatz von geteilten elektrochemischen Zellen, von geringen Stromdichten (< 1 A/dm²) und das Auftreten von Ausbeute verringernden Nebenreaktionen, vermieden werden. Diese Aufgabe wird gelöst durch die im Folgenden beschriebenen und durch die beanspruchten Ausführungsformen.

Entsprechend betrifft die vorliegende Erfindung Verfahren zur elektrochemischen Herstellung von γ-Hydroxycarbonsäureestern und/oder γ-Lactonen durch reduktive Kreuzkopplung von α,β-ungesättigten Estern mit Carbonylverbindungen in einer ungeteilten Elektrolysezelle, dadurch gekennzeichnet, dass das Kathodenmaterial ausgewählt ist aus der Gruppe bestehend aus Blei, Bleilegierungen, Kadmium, Kadmiumlegierungen, Quecksilber, Stahl, Glaskohlenstoff und Bor-dotierte Diamanten, und dass ein basischer, wässriger Elektrolyt mit mindestens einem Leitsalz, ausgewählt aus bisquartären und multiquartären Ammonium- und Phosphoniumsalzen, eingesetzt wird.

Unter einer Carbonylverbindung ist im Rahmen dieser Erfindung ein Aldehyd oder ein Keton, bevorzugt ein Aldehyd, zu verstehen. Die erfindungsgemäßen Carbonylverbindungen weisen vorzugsweise eine geringe Löslichkeit in Wasser von weniger als 100 g/l, bevorzugt weniger als 50 g/L, besonders bevorzugt weniger als 30 g/l auf, jeweils bei 20°C. Bevorzugt sind an der Carbonylgruppe der Carbonylverbindungen Alkyl- und/oder Aryl-Gruppen gebunden, die auch weitere funktionelle Gruppen (bspw. Alkohol-, Ether-, Carbonyl-, Carbonsäuregruppen usw.) enthalten können, und die durch Sauerstoff, Schwefel oder Stickstoff unterbrochene Alky-, Alkylen- oder Arylengruppen sein können. Besonders bevorzugt sind aliphatische Carbonylverbindungen, die neben der Carbonylgruppe keine weiteren Hetereoatome aufweisen. Geeignete Carbonylverbindungen sind beispielsweise Pentanal, 2-Methylpentanal, Hexanal, 2-Ethylhexanal, Heptanal, 4-Formyltetrahydropyran, 4-Methoxybenzaldehyd, 4-tert-Butylbenzaldehyd, 4-Methylbenzaldehyd, Glyoxal, Glutaraldehyd, Methylglyoxal, Cyclohexenon, Cyclohexanon, Diethylketon. Besonders bevorzugte Carbonylverbindungen sind Pentanal, 2-Methylpentanal, Hexanal und Heptanal.

Unter einem α,β-ungesättigten Ester im Rahmen dieser Erfindung sind Acrylsäureester-Derivate zu verstehen, die an Position 2 und 3 unabhängig von einander substituiert sein können, wobei an Position 3 auch zwei Substituenten möglich sind. Bei den Substituenten handelt es sich bevorzugt um Alkylgruppen, Halogenatome, C1-C20-Alkoxygruppen, durch Sauerstoff, Schwefel oder Stickstoff unterbrochene Alkyl-, Alkylen oder Arylenreste, Nitrilgruppen sowie Nitrogruppen. Bevorzugt sind die Substituenten aus der Gruppe Methyl, Ethyl, n-Propyl, Isopropyl, n-Butyl, tert-Butyl, Trifluormethyl, Fluor, Chlor, Brom, Iod, Methoxy, Ethoxy, Methylen, Ethylen, Propylen, Isopropylen, Benzyliden, Nitril und Nitro. Besonders bevorzugt sind Substituenten aus der Gruppe Methyl, Ethyl, Methoxy, Ethoxy. Bei dem α,β-ungesättigten Ester handelt es sich bevorzugt um einen C1-C12-Alkylester, besonders bevorzugt um einen C1-C5-Alkyl-Ester, ganz bevorzugt um einen Methyl- oder Ethylester. Die erfindungsgemäßen α,β-ungesättigten Ester weisen vorzugsweise eine geringe Löslichkeit in Wasser von weniger als 100 g/l, bevorzugt weniger als 50 g/l, besonders bevorzugt weniger als 20 g/l auf, jeweils bei 20°C.

α,β-ungesättigte Ester und Carbonylverbindungen sind die Edukte der erfindungsgemäßen reduktiven Kopplung.

Ein wässriger Elektrolyt im Sinn dieser Erfindung enthält neben den Edukten noch Wasser, mindestens ein Leitsalz und mindestens einen Puffer als Komponenten. Vorzugsweise enthält der Elektrolyt darüber hinaus auch noch mindestens einen Komplexbildner und/oder mindestens einen Anodenkorrosionsinhibitor als weitere Komponenten. Der wässrige Elektrolyt in seiner Gesamtheit mit allen Komponenten inklusive der Edukte wird im Folgenden auch Reaktionselektrolyt genannt. Die wässrige Zusammensetzung, die dem Reaktionselektrolyten ohne Edukte entspricht, wird im Folgenden auch Supportelektrolyt genannt. Der wässrige Reaktionselektrolyt zeichnet sich aus durch einen Wasseranteil von vorzugsweise mindestens 20 Gew.- %, besonders bevorzugt mindestens 50 Gew.-%, insbesondere mindestens 75 Gew.-% bezogen auf den gesamten wässrigen Reaktionselektrolyten.

Der erfindungsgemäße Reaktionselektrolyt enthält mindestens ein Leitsalz, ausgewählt aus bisquartären und multiquartären Ammonium- und Phosphoniumsalzen, das die kathodische Wasserstoffbildung unterdrückt. Bevorzugt werden außer diesen bisquartären und multiquartären Ammonium- und Phosphoniumsalzen keine weiteren Leitsalze eingesetzt. Im Allgemeinen setzt man das Leitsalz in einer Menge im Bereich 0,01 bis 2,5 Gew.-%, bevorzugt von 0,01 bis 1,5 Gew.-%, bevorzugt von 0,01 bis 0,5 Gew.-%, besonders bevorzugt von 0,05 bis 0,25 Gew.-% bezogen auf den gesamten wässrigen Reaktionselektrolyten ein. Besonders geeignete Leitsalze sind bisquartäre Ammonium- und Phosphoniumsalze (EP 635587 A). Besonders bevorzugt ist der Einsatz von Bis(dibutylethyl)hexamethylendiamoniumhydroxid als Leitsalz für den Elektrolyten. Als Gegenionen kommen beispielsweise Sulfat, Hydrogensulfat, Alkylsulfate, Arylsulfate, Alkylsulfonate, Arylsulfonate, Halogenide, Phosphate, Carbonate, Alkylphosphate, Alkylcarbonate, Nitrat, Alkoholate, Hydroxid, Tetrafluorborat oder Perchlorat in Betracht. Weiterhin kommen die von den vorstehend genannten Anionen abgeleiteten Säuren als Leitsalze in Betracht, also zum Beispiel Schwefelsäure, Sulfonsäuren sowie Carbonsäuren. Daneben eignen sich als Leitsalze auch ionische Flüssigkeiten. Geeignete ionische Flüssigkeiten sind beschrieben in "Ionic Liquids in Synthesis", Hrsg. Peter Wasserscheid, Tom Welton, Verlag Wiley VCH, 2003, Kap. 1 bis 3 sowie in der DE 102004011427 A.

Des Weiteren enthält der Reaktionselektrolyt mindestens einen Puffer mit einem Pufferbereich bei einem pH-Wert von 7 bis 11, bevorzugt von 8 bis 10, zur Pufferung der bei der anodischen Sauerstoffbildung entstehenden Protonen. Geeignete Puffer sind beispielsweise Hydrogenphosphat oder Hydrogencarbonat, bevorzugt in Form ihrer Natriumsalze. Besonders bevorzugt ist der Einsatz von Dinatriumhydrogenphosphat als Puffer für den Elektrolyten. Im Allgemeinen setzt man den Puffer in einer Menge im Bereich von 0,9 bis 8 Gew.-%, vorzugsweise von 4 bis 7 Gew.-% bezogen auf den gesamten wässrigen Reaktionselektrolyten ein.

Ferner enthält der Reaktionselektrolyt vorzugsweise einen oder mehrere Anodenkorrosionsinhibitoren wie die für diese Zwecke bekannten Borate, bevorzugt Dinatriumdiborat und Orthoborsäure, in einer Menge im Bereich von 0,4 bis 3 Gew.-%, bevorzugt von 1 bis 2 Gew.-% bezogen auf den gesamten wässrigen Reaktionselektrolyten.

Ferner enthält der Reaktionselektrolyt vorzugsweise einen oder mehrere Komplexbildner, um die Ausfällung von Eisen- und Blei-Ionen zu verhindern. Beispielhaft seien genannt Ethylendiamintetraacetat (EDTA), Triethanolamin (TEA), Triethylamin, Nitrilotriacetat, bevorzugt EDTA in einer Menge im Bereich von 0 bis 1 Gew. %, vorzugsweise 0,1 bis 0,5 Gew. % bezogen auf den gesamten wässrigen Reaktionselektrolyten, und/oder TEA in einer Menge im Bereich von 0 bis 0,5 Gew. %, vorzugsweise 0,05 bis 0,2 Gew. % bezogen auf den gesamten wässrigen Reaktionselektrolyt. Anstelle von TEA kann auch Triethylamin in einer Menge von 0 bis 0,5 Gew. %, vorzugsweise 0,05 bis 0,2 Gew. % bezogen auf den gesamten wässrigen Reaktionselektrolyt verwendet werden.

Als Anodenmaterial kann man bekannte Anodenmaterialien verwenden, üblicherweise werden bei ungeteilten Zellen Materialien mit geringer Sauerstoffüberspannung eingesetzt, beispielsweise Kohlenstoffstahl, Glaskohlenstoff, Stahl, Quecksilber, Kadmium, Platin, Eisen, Nickel, Magnetit, Blei, Bleilegierungen oder Bleidioxid. Bevorzugt wird eine Anode aus Stahl, Eisen, Blei oder einer Bleilegierung eingesetzt.

Als Kathoden werden Blei, Bleilegierungen, Kadmium, Kadmiumlegierungen, Quecksilber, Stahl, Glaskohlenstoff oder Bor-dotierte Diamantelektroden eingesetzt. Bevorzugte werden als Kathodenmaterialien Blei, Bleilegierungen, Kadmium, Stahl und Glaskohlenstoff eingesetzt. Besonders bevorzugt werden als Kathodenmaterialien Blei und Bleilegierungen eingesetzt.

In dem erfindungsgemäßen wässrigen Reaktionselektrolyten liegen die organischen Edukte (α,β-ungesättigter Ester und Carbonylverbindungen) sowie die entstehenden Produkte (γ-Hydroxycarbonsäureestern und γ-Lactonen) als organische Phase einer Emulsion vor. Die Aufrechterhaltung der Emulsion während der Elektrolyse kann gewährleistet werden durch mechanische Agitation wie Rühren oder Umpumpen des Elektrolyten in der Elektrolysezelle, oder aber auch durch Zusatz geeigneter die Emulsion stabilisierender Emulgatoren. Bevorzugt wird die Emulsion während der Elektrolyse aufrechterhalten durch mechanische Agitation wie Rühren oder Umpumpen des Elektrolyten. Nach erfolgter Elektrolyse kann die Entmischung der Emulsion erreicht werden, beispielsweise durch Beendigung der Agitation oder durch Zusatz eines geeigneten Flockungsmittels. Nach Entmischung der Emulsion in eine wässrige und eine organische Phase können die Produkte sowie ggf. unumgesetzte Edukte leicht mit der organischen Phase von dem wässrigen Elektrolyten abgetrennt werden. Dadurch wird die Abtrennung der Produkte aus dem Elektrolyten vereinfacht.

Bei der erfindungsgemäßen Elektrolyse werden die Edukte α,β-ungesättigter Ester und Carbonylverbindungen vorzugsweise in im Wesentlichen äquimolaren Verhältnis eingesetzt. Üblicherweise liegt das molare Verhältnis von eingesetztem α,β-ungesättigter Ester zu eingesetzter Carbonylverbindung zwischen 0,25 und 4, bevorzugt zwischen 0,5 und 2, besonders bevorzugt zwischen 0,8 und 1,2. Während nach den bisher bekannten Verfahren zur reduktiven Kopplung von α,β-ungesättigten Ester mit Carbonylverbindungen ein Überschuss an Carbonylverbindung eingesetzt wird, um die homo-Kopplung des Esters zu unterdrücken, weist das erfindungsgemäße Verfahren eine hohe Selektivität auf zugunsten des Kreuzkopplungsproduktes aus α,β-ungesättigtem Ester und Carbonylverbindung. Bei Einsatz der Edukte in im Wesentlichen äquimolarem Verhältnis lassen sich mit dem erfindungsgemäßen Verfahren besonders gute Ausbeuten für das Kreuzkopplungsprodukt realisieren. Vorzugsweise wird der α,β-ungesättigter Ester eingesetzt in einer Menge von 1 bis 25 Gew.-%, besonders bevorzugt von 5 bis 10 Gew.-% bezogen auf den gesamten wässrigen Reaktionselektrolyten.

Die Elektrolyse wird üblicherweise bei einer Stromdichte von mindestens 1 A/dm², vorzugsweise bei 1 bis 4 A/dm² durchgeführt. Es ist aber auch möglich, die Elektrolyse bei einer höheren Stromdichte von bis zu 20 A/dm² durchzuführen.
Die erfindungsgemäße Elektrolyse wird üblicherweise bei einer Temperatur von 20 bis 60°C und unter Normaldruck durchgeführt.

Die Elektrolyse kann sowohl kontinuierlich als auch diskontinuierlich und in allen üblichen ungeteilten Elektrolysezellen, wie beispielsweise in Becherglas- oder Platten- und Rahmenzellen oder Zellen mit Festbett- oder Fließbettelektroden, durchgeführt werden. Es ist sowohl die monopolare als auch die bipolare Schaltung der Elektroden anwendbar. Bevorzugt wird der Elektrolyt in der Elektrolysezelle umgepumpt oder gerührt, wodurch auch dessen Vorliegen als Emulsion aufrecht erhalten werden kann.
Ganz besonders geeignet sind bipolar geschaltete Kapillarspaltzellen oder Plattenstapelzellen, bei denen die Elektroden als Platten ausgestaltet sind und planparallel angeordnet sind (Ull-mann's Encyclopedia of Industrial Chemistry, 2009 electronic release, VCH-Verlag Weinheim, Volume Electrochemistry, Chapter 3. Electrochemical Cells sowie Chapter 5, Organic Electrochemistry, Subchapter 5.4.3. Electrochemical Cells).
Bei einer ungeteilten Elektrolysezelle sind Anoden- und Kathodenraum nicht durch eine Membran voneinander getrennt. Solche ungeteilten Zellen sind günstiger und technisch leichter zu realisieren. Insbesondere bei organischen Verfahren kommt es bei der Verwendung von geteilten Zellen zu einer schnellen Alterung der Membran, was unbefriedigenden Standzeiten zur Folge hat.
Bei dem erfindungsgemäßen Verfahren zur elektrochemischen reduktiven Kreuzkopplung von α,β-ungesättigten Estern mit Carbonylverbindungen kann das γ-Lacton oder der entsprechende γ-Hydroxycarbonsäureester jeweils allein oder als Gemisch entstehen. Bei Bedarf kann etwaig entstehender γ-Hydroxycarbonsäureester im Anschluss an die elektrochemische reduktive Kreuzkopplung durch Umesterung zum γ-Lacton umgesetzt werden. Die Umesterung zum γ-Lacton kann beispielsweise durch Erwärmen des γ-Hydroxycarbonsäureesters in Gegenwart von Säure erfolgen. Falls nötig kann dem Reaktionsgemisch der dabei freigesetzte Alkohol entzogen werden, um die Reaktion in Richtung des γ-Lactons zu verschieben. Umgekehrt kann bei Bedarf etwaig entstehendes γ-Lacton im Anschluss an die elektrochemische reduktive Kreuzkopplung durch Umesterung (Alkoholyse) zum γ-Hydroxycarbonsäureester umgesetzt werden, beispielsweise durch Erwärmen des γ-Lactons in alkalischen, nicht-wässrigen alkoholischen Lösungen. Der γ-Hydroxycarbonsäureester kann im Anschluss daran durch Verseifung weiter umgesetzt werden zur freien Säure oder zum Carbonsäuresalz. Dazu wird der γ-Hydroxycarbonsäureester beispielsweise mit wässriger, alkalischer Lösungen erwärmt. Alternativ kann die freie γ-Hydroxycarbonsäure bzw. deren Salz auch direkt aus dem γ-Lacton durch Verseifen hergestellt werden. Dies kann beispielsweise erfolgen durch Erwärmen des γ-Lactons in wässriger, alkalischer Lösungen.

Ein weiterer Gegenstand der Anmeldung sind die nach dem erfindungsgemäßen Verfahren herstellbaren γ-Butyrolacton-Derivate der allgemeinen Formel I 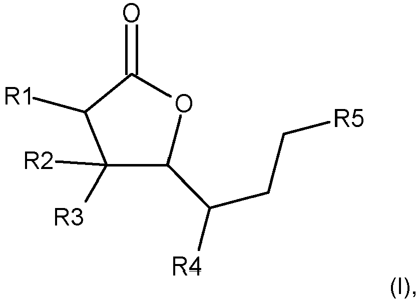 wobei
R1, R2 und R3 jeweils unabhängig ein Wasserstoff oder eine Alkyl-Gruppe mit 1 bis 5 C-Atomen, bevorzugt ein Wasserstoff, eine Methyl- oder Ethylgruppe darstellen, und
R4 und R5 Alkyl-Gruppen mit 1 bis 4 C-Atomen, bevorzugt 1 bis 3 C-Atomen darstellen, wobei R4 und R5 identische Reste darstellen.
Die Verbindungen der Formel I können hergestellt werden durch die erfindungsgemäße elektrochemischen Kreuzkupplung von α,β-ungesättigten Estern der Formel II 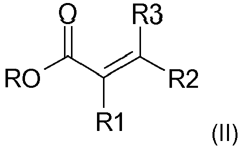 mit 2-Alkyl-Alkanalen der Formel III 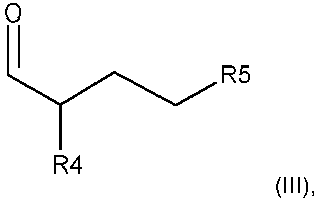 wobei R1 bis R5 die gleiche Bedeutung haben wie bei den Verbindungen der Formel I und R eine Alkyl-Gruppe darstellt, üblicherweise eine Alkyl-Gruppe mit 1 bis 12 C-Atomen, bevorzugt mit 1 bis 5 C-Atomen, ganz besonders bevorzugt eine Methyl- oder Ethyl-Gruppe.

Bevorzugter Gegenstand der Anmeldung sind die nach dem erfindungsgemäßen Verfahren herstellbaren γ-Butyrolacton-Derivate der allgemeinen Formel IV 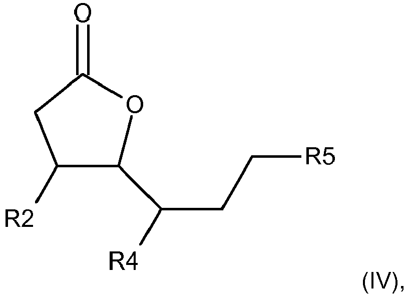 wobei
R2 ein Wasserstoff oder eine Alkyl-Gruppe mit 1 bis 5 C-Atomen, bevorzugt ein Wasserstoff, eine Methyl- oder eine Ethylgruppe darstellt, und R4 und R5 Alkyl-Gruppen mit 1 bis 4 C-Atomen, bevorzugt 1 bis 3 C-Atomen darstellen, wobei R4 und R5 identische Reste darstellen. Die Verbindungen der Formel IV können hergestellt werden durch die erfindungsgemäße elektrochemischen Kreuzkupplung von α,β-ungesättigten Estern der Formel II (mit R1 und R3 jeweils als Wasserstoff) mit 2-Alkyl-Alkanalen der Formel III.

Die 2-Alkyl-Alkanale der Formel III können beispielsweise durch Aldolkondensation von Alkanalen mit 3 bis 6 C-Atomen (Propanal, Butanal, Pentanal, bzw. Hexanal) hergestellt werden. Besonders bevorzugt sind die γ-Butyrolacton-Derivate 4-(2-Pentyl)butyrolacton 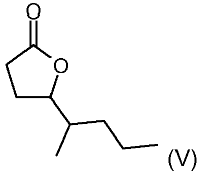 und 3-Methyl-4-(2-pentyl)butyrolacton 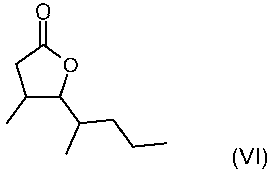 die hergestellt werden können durch die erfindungsgemäße elektrochemische Kreuzkupplung von Acrylsäureester bzw. Crotonsäureester mit 2-Methylpentanal.

Ein weiterer Gegenstand der Anmeldung sind die nach dem erfindungsgemäßen Verfahren herstellbaren γ-Hydroxycarbonsäuren bzw. γ-Hydroxycarbonsäureester der allgemeinen Formel VIII 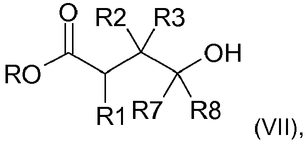 wobei
R1, R2, R3 und R7 jeweils unabhängig ein Wasserstoff oder eine Alkyl-Gruppe mit 1 bis 5 C-Atomen, bevorzugt ein Wasserstoff, eine Methyl- oder Ethylgruppe darstellen,
R ein Wasserstoff oder eine Alkyl-Gruppe darstellt, üblicherweise ein Wasserstoff oder eine Alkyl-Gruppe mit 1 bis 5 C-Atomen, und R8 eine verzweigte Alkyl-Gruppe mit 3 bis 10 C-Atomen darstellt.

Bevorzugter Gegenstand der Anmeldung sind die nach dem erfindungsgemäßen Verfahren herstellbaren γ-Hydroxycarbonsäuren bzw. γ-Hydroxycarbonsäureester der allgemeinen Formel VIII, wobei R1, R2 und R3 jeweils unabhängig ein Wasserstoff oder eine Alkyl-Gruppe mit 1 bis 5 C-Atomen, bevorzugt ein Wasserstoff, eine Methyl- oder Ethylgruppe darstellen, R ein Wasserstoff oder eine Alkyl-Gruppe darstellt, üblicherweise ein Wasserstoff oder eine Alkyl-Gruppe mit 1 bis 5 C-Atomen, R7 ein Wasserstoff darstellt, und R8 eine verzweigte Alkyl-Gruppe mit 3 bis 10 C-Atomen darstellt.

Die Verbindungen der Formel VIII können hergestellt werden durch die erfindungsgemäße elektrochemischen Kreuzkupplung von α,β-ungesättigten Estern der Formel II mit der Carbonylverbindung der Formel VIII 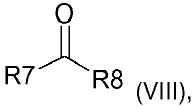 wobei R7 und R8 die gleiche Bedeutung haben wie bei den Verbindungen der Formel VIII.

Alkyl-Gruppen im Sinne der Anmeldung können grundsätzlich sowohl verzweigt wie unverzweigt, sowohl linear wie cyclisch und sowohl gesättigt wie ungesättigt (auch mehrfach ungesättigt) sein. Vorzugsweise weisen 1 bis 20, besonders bevorzugt 1 bis 6 C-Atome auf. Vorzugsweise weisen sie keine Heteroatome auf.

Aryl-Gruppen im Sinne der Anmeldung sind aromatische Reste mit vorzugsweise 5 bis 20 C-Atomen.

Ein weiterer Gegenstand der Anmeldung ist die Verwendung der erfindungsgemäßen γ-Butyrolacton-Derivate der Formel I, vorzugsweise die γ-Butyrolacton-Derivate der Formel IV, besonders bevorzugt 4-(2-Pentyl)butyrolacton oder 3-Methyl-4-(2-pentyl)butyrolacton als Riech- oder Aromastoffe. 4-(2-Pentyl)butyrolacton hat ein birnenähnliches und 3-Methyl-4-(2-pentyl)butyrolacton ein holzähnliches Aroma.

### Ausführungsbeispiele

Die Erfindung wird nun durch die nachfolgenden, nichtlimitierenden Beispiele näher erläutert.

### Beispiel 1

Elektrochemische Darstellung von γ-Hydroxypelargonsäureethylester und 4-Pentylbutyrolacton durch reduktive Kreuzkopplung von Acrylsäureethylester mit Hexanal bei Einsatz von Hexanal im Überschuss

Acrylsäureethylester (1,7 Gew.-%) und Hexanal (29,7 Gew.-%) wurden in einem wässrigen Elektrolyten (0,16 Gew.-% Bis(dibutylethyl)hexamethylendiamoniumhydroxid (Bisquat), 0,38 Gew.-% EDTA, 0,14 Gew.-% TEA, 1,45 Gew.-% Na₂B₄O₇ und 5,84 Gew.-% Na₂HPO₄ in Wasser bei pH von 10) emulgiert (alle Gew.-%-Angaben beziehen sich auf den gesamten wässrigen Reaktionselektrolyt) und bei einer Stromdichte von 2,23 A/dm² und einer Temperatur von 20 °C in einer Topfzelle der galvanostatischen Elektrolyse unterworfen. Der Stromdurchsatz betrug 2 F/mol Ester. Als Elektroden wurden eine Stahlanode und eine Bleikathode verwendet (Elektrodenfläche von 0,1 dm² und Abstand von 1 cm). Zur Überprüfung der Reaktion wurde der Methyltributylammoniummethylsulfat-Extrakt (MTBE-Extrakt) einer Probe des Elektrolyseaustrags gaschromatographisch untersucht. Es wurde für das 4-Pentylbutyrolacton eine Ausbeute von 0,1 % und für den entsprechenden γ-Hydroxypelargonsäureethylester von 2,9 % erzielt. Das entsprach einer Gesamtausbeute an Zielprodukten von 3,0 % der theoretischen Ausbeute.

### Beispiel 2

Elektrochemische Darstellung von γ-Hydroxypelargonsäureethylester und 4-Pentylbutyrolacton durch reduktive Kreuzkopplung von Acrylsäureethylester mit Hexanal bei Einsatz der Edukte in äquimolarem Verhältnis

Acrylsäureethylester (5,9 Gew.-%) und Hexanal (6,0 Gew.-%) wurden umgesetzt (alle Gew.-%-Angaben beziehen sich auf den gesamten wässrigen Reaktionselektrolyt) und analysiert, wie in Beispiel 1 beschrieben. Es wurde für das 4-Pentylbutyrolacton eine Ausbeute von 23,7 % und für den entsprechenden γ-Hydroxypelargonsäureethylester von 48,0 % erzielt. Das entsprach einer Gesamtausbeute an Zielprodukten von 71,7 % der theoretischen Ausbeute.

### Vergleichsbeispiel 1

Elektrochemische Darstellung von γ-Hydroxypelargonsäureethylester und 4-Pentylbutyrolacton durch reduktive Kreuzkopplung von Acrylsäureethylester mit Hexanal bei Einsatz von Hexanal im Überschuss

Entsprechend der von Bürger beschriebenen reduktiven Kopplung wurden Acrylsäureethylester (1,7 Gew.-%) und Hexanal (29,7 Gew.-%) in einem Elektrolyten (17,0 Gew.-% Tetrabutylamintetrafluorborat (Bu₄NBF₄) in einem 3:1-Gemisch aus Dioxan und Ethanol) gelöst (alle Gew.-%-Angaben beziehen sich auf den gesamten wässrigen Reaktionselektrolyt) und bei einer Stromdichte von zunächst 2,23 A/dm² und einer Temperatur von 21 °C in einer Topf-Elektrolysezelle der galvanostatischen Elektrolyse unterworfen. Der Stromdurchsatz betrug 2 F/mol Ester. Im Verlauf der Elektrolyse sank die Stromdichte auf 0,73 A/dm². Als Elektroden wurden eine Platinanode und eine Graphitkathode verwendet (Elektrodenfläche von 0,1 dm² und Abstand von 1 cm). Zur Überprüfung der Reaktion wird der Methyltributylammoniummethylsulfat-Extrakt einer Probe des Elektrolyseaustrags gaschromatographisch untersucht. Es wurde für das 4-Pentylbutyrolacton eine Ausbeute von 2,0 % und von dem entsprechenden γ-Hydroxypelargonsäureethylester von 0,2 % erzielt. Das entsprach einer Gesamtausbeute an Zielprodukten von 2,2 % der theoretischen Ausbeute.

### Vergleichsbeispiel 2

Elektrochemische Darstellung von γ-Hydroxypelargonsäureethylester und 4-Pentylbutyrolacton durch reduktive Kreuzkopplung von Acrylsäureethylester mit Hexanal bei Einsatz der Edukte in äquimolarem Verhältnis

Acrylsäureethylester (5,9 Gew.-%) und Hexanal (6,0 Gew.-%) wurden bei 22°C umgesetzt (alle Gew.-%-Angaben beziehen sich auf den gesamten wässrigen Reaktionselektrolyt) und anschließend analysiert, wie in Vergleichsbeispiel 1 beschrieben, wobei die Stromdichte während der Versuchsdurchführung konstant blieb. Es wurde für das 4-Pentylbutyrolacton eine Ausbeute von 16,5 % und für den entsprechenden γ-Hydroxypelargonsäureethylester von 0,7 % erzielt. Das entsprach einer Gesamtausbeute an Zielprodukten von 17,2 % der theoretischen Ausbeute.

### Beispiel 3

### Darstellung von Whiskeylacton

Crotonsäureethylester (6,9 Gew.-%) und Pentanal (5,2 Gew.-%) wurden in einem wässrigen Elektrolyten (0,16 Gew.-% Bis(dibutylethyl)hexamethylendiamoniumhydroxid (Bisquat), 0,38 Gew.-% EDTA, 0,14 Gew.-% TEA, 1,45 Gew.-% Na₂B₄O₇ und 5,84 Gew.-% Na₂HPO₄ in Wasser bei pH von 10) emulgiert (alle Gew.-%-Angaben beziehen sich auf den gesamten wässrigen Reaktionselektrolyt) und bei einer Stromdichte von 2,23 A/dm² und einer Temperatur von 25 °C in einer Rahmen-Elektrolysezelle der galvanostatischen Elektrolyse unterworfen. Der Stromdurchsatz betrug 2 F/mol Ester. Als Elektroden wurden eine Stahlanode und eine Bleikathode verwendet (Elektrodenfläche von 0,1 dm² und Abstand von 1 cm). Zur Überprüfung der Reaktion wird der MTBE-Extrakt einer Probe des Elektrolyseaustrags gaschromatographisch untersucht. Es wurde für das 3-Methyl-4-butyl-butyrolacton (Whiskeylacton) eine Ausbeute von 68,5 % erzielt und von 24,2 % für den entsprechenden γ-Hydroxycarbonsäureethylester. Das entsprach einer Gesamtausbeute an Zielprodukten von 92,7 % der theoretischen Ausbeute.

### Beispiel 4

### Darstellung von 4-(2-Pentyl)butyrolacton

Acrylsäureethylester (5,9 Gew.-%) und 2-Methylpentanal (6,0 Gew.-%) wurden entsprechend Beispiel 3 elektrochemisch umgesetzt (alle Gew.-%-Angaben beziehen sich auf den gesamten wässrigen Reaktionselektrolyt). Dabei wurde für das 4-(2-Pentyl)butyrolacton eine Ausbeute von 88,4 % erzielt.

### Beispiel 5

### Darstellung von 3-Methyl-4-(2-pentyl)butyrolacton

Crotonsäureethylester (6,7 Gew.-%) und 2-Methylpentanal (5,9 Gew.-%) wurden entsprechend Beispiel 3 elektrochemisch umgesetzt (alle Gew.-%-Angaben beziehen sich auf den gesamten wässrigen Reaktionselektrolyt). Dabei wurde für das 4-(2-Pentyl)butyrolacton eine Ausbeute von 66,7 % erzielt und von 26,6 % für die entsprechende γ-Hydroxycarbonsäureethylester. Das entsprach einer Gesamtausbeute an Zielprodukten von 93,2 % der theoretischen Ausbeute.

### Beispiel 6

### Darstellung von 3,3-Dimethyl-4-pentylbutyrolacton

3,3-Dimethylacrylsäuremethylester (4,9 Gew.-%) und Hexanal (4,3 Gew.-%) wurden entsprechend Beispiel 3 elektrochemisch umgesetzt (alle Gew.-%-Angaben beziehen sich auf den gesamten wässrigen Reaktionselektrolyt). Dabei wurde für das 3,3-Dimethyl-4-pentylbutyrolacton eine Ausbeute von 37,2 % erzielt.

### Beispiel 7

### Darstellung von 2-Methyl-4-butylbutyrolacton

Methacrylsäureethylester (6,7 Gew.-%) und Pentanal (5,2 Gew.-%) wurden entsprechend Beispiel 3 elektrochemisch umgesetzt (alle Gew.-%-Angaben beziehen sich auf den gesamten wässrigen Reaktionselektrolyt). Dabei wurde für das 2-Methyl-4-butylbutyrolacton eine Ausbeute von 81,0 % erzielt.

### Beispiel 8

### Darstellung von 2-Methyl-4-(2-pentyl)butyrolacton

Methacrylsäureethylester (6,7 Gew.-%) und Methylpentanal (5,9 Gew.-%) wurden entsprechend Beispiel 3 elektrochemisch umgesetzt (alle Gew.-%-Angaben beziehen sich auf den gesamten wässrigen Reaktionselektrolyt). Dabei wurde für das 2-Methyl-4-(2-pentyl)butyrolacton eine Ausbeute von 70,9 % erzielt.

## Patentansprüche

1. Verfahren zur elektrochemischen Herstellung von γ-Hydroxycarbonsäureestern und/oder γ-Lactonen durch reduktive Kreuzkopplung von α,β-ungesättigten Estern mit Carbonylverbindungen in einer ungeteilten Elektrolysezelle, **dadurch gekennzeichnet, dass** das Kathodenmaterial ausgewählt ist aus der Gruppe bestehend aus Blei, Bleilegierungen, Kadmium, Kadmiumlegierungen, Quecksilber, Stahl, Glaskohlenstoff und Bor-dotierte Diamanten, und dass ein basischer, wässriger Elektrolyt mit mindestens einem Leitsalz, ausgewählt aus bisquartären und multiquartären Ammonium- und Phosphoniumsalzen, eingesetzt wird.

2. Das Verfahren gemäß Anspruch 1, **dadurch gekennzeichnet, dass** die α,β-ungesättigten Ester mit den Carbonylverbindungen in einem im Wesentlichen äquimolaren Verhältnis eingesetzt werden.

3. Das Verfahren gemäß Anspruch 1 oder 2, **dadurch gekennzeichnet, dass** die Edukte α,β-ungesättigte Ester und Carbonylverbindungen sowie die Produkte der reduktiven Kreuzkopplung dieser Edukte als Emulsion in dem wässrigen Elektrolyten vorliegen.

4. Das Verfahren gemäß Anspruch 1 bis 3, **dadurch gekennzeichnet, dass** das Leitsalz ein bisquartäres Ammonium- oder Phosphoniumsalz ist.

5. Das Verfahren gemäß einem der Ansprüche 1 bis 4, **dadurch gekennzeichnet, dass** das Kathodenmaterial ausgewählt ist aus der Gruppe bestehend aus Blei, Bleilegierungen, Kadmium, Stahl und Glaskohlenstoff.

6. Das Verfahren gemäß einem der Ansprüche 1 bis 5, **dadurch gekennzeichnet, dass** das Anodenmaterial ausgewählt ist aus der Gruppe bestehend aus Kohlenstoffstahl, Glaskohlenstoff, Stahl, Quecksilber, Kadmium, Platin, Eisen, Nickel, Magnetit, Blei, Bleilegierungen oder Bleidioxid.

7. Das Verfahren gemäß einem der Ansprüche 1 bis 6, **dadurch gekennzeichnet, dass** der wässrige Elektrolyt mindestens einen Puffer mit einem Pufferbereich bei einem pH-Wert von 7 bis 11 enthält.

8. Das Verfahren gemäß einem der Ansprüche 1 bis 7, **dadurch gekennzeichnet, dass** der wässrige Elektrolyt zusätzlich einen oder mehrere Anodenkorrosionsinhibitoren enthält.

9. Das Verfahren gemäß einem der Ansprüche 1 bis 8, **dadurch gekennzeichnet, dass** der wässrige Elektrolyt zusätzlich einen oder mehrere Komplexbildner enthält.

10. Das Verfahren gemäß einem der Ansprüche 1 bis 9, **dadurch gekennzeichnet, dass** das molare Verhältnis von eingesetztem α,β-ungesättigter Ester zu eingesetzter Carbonylverbindung zwischen 0,25 und 4 liegt.

11. Das Verfahren gemäß einem der Ansprüche 1 bis 10, **dadurch gekennzeichnet, dass** die Carbonylverbindung ein Aldehyd ist.

12. Das Verfahren gemäß einem der Ansprüche 1 bis 11, **dadurch gekennzeichnet, dass** entstehender γ-Hydroxycarbonsäureester in einem anschließenden Verfahrensschritt zum γ-Lacton umgeestert wird.

13. Das Verfahren gemäß einem der Ansprüche 1 bis 11, **dadurch gekennzeichnet, dass** entstehendes γ-Lacton in einem anschließenden Verfahrensschritt zum γ- Hydroxycarbonsäureester umgeestert wird.

14. Das Verfahren gemäß einem der Ansprüche 1 bis 11, **dadurch gekennzeichnet, dass** entstehendes γ-Lacton und/oder entstehender γ-Hydroxycarbonsäureester in einem anschließenden Verfahrensschritt zur γ-Hydroxycarbonsäure verseift wird.

## Claims

1. A process for the electrochemical preparation of γ-hydroxycarboxylic esters and/or γ-lactones by reductive cross-coupling of α,β-unsaturated esters with carbonyl compounds in an undivided electrolysis cell, wherein the cathode material is selected from the group consisting of lead, lead alloys, cadmium, cadmium alloys, mercury, steel, glassy carbon and boron-doped diamonds and a basic, aqueous electrolyte comprising at least one electrolyte salt selected from among bisquaternary and multiquaternary ammonium and phosphonium salts is used

2. The process according to claim 1, wherein the α,β-unsaturated esters are used in an essentially equimolar ratio with the carbonyl compounds.

3. The process according to claim 1 or 2, wherein the starting materials α,β-unsaturated esters and carbonyl compounds and the products of the reductive cross-coupling of the starting materials are present as an emulsion in the aqueous electrolyte.

4. The process according to claim 1 to 3, wherein the electrolyte salt is a bisquaternary ammonium or phosphonium salt.

5. The process according to any of claims 1 to 4, wherein the cathode material is selected from the group consisting of lead, lead alloys, cadmium, steel and glassy carbon.

6. The process according to any of claims 1 to 5, wherein the anode material is selected from the group consisting of carbon steel, glassy carbon, steel, mercury, cadmium, platinum, iron, nickel, magnetite, lead, lead alloys and lead dioxide.

7. The process according to any of claims 1 to 6, wherein the aqueous electrolyte comprises at least one buffer having a buffering range at a pH of from 7 to 11.

8. The process according to any of claims 1 to 7, wherein the aqueous electrolyte additionally comprises one more anode corrosion inhibitors.

9. The process according to any of claims 1 to 8, wherein the aqueous electrolyte additionally comprises one or more complexing agents.

10. The process according to any of claims 1 to 9, wherein the molar ratio of α,β-unsaturated ester and carbonyl compound used is in the range from 0.25 to 4.

11. The process according to any of claims 1 to 10, wherein the carbonyl compound is an aldehyde.

12. The process according to any of claims 1 to 11, wherein γ-hydroxycarboxylic ester formed is transesterified to γ-lactone in a subsequent process step.

13. The process according to any of claims 1 to 11, wherein γ-lactone formed is transesterified to the γ-hydroxycarboxylic ester in a subsequent process step.

14. The process according to any of claims 1 to 11, wherein γ-lactone formed and/or γ-hydroxycarboxylic ester formed is hydrolyzed to the γ-hydroxycarboxylic acid in a subsequent process step.

## Revendications

1. Procédé de fabrication électrochimique d'esters d'acides γ-hydroxycarboxyliques et/ou de γ-lactones par couplage croisé réducteur d'esters α,β-insaturés avec des composés de carbonyle dans une cellule d'électrolyse non divisée, **caractérisé en ce que** le matériau de la cathode est choisi dans le groupe constitué par le plomb, les alliages de plomb, le cadmium, les alliages de cadmium, le mercure, l'acier, le carbone vitreux et les diamants dopés au bore, et **en ce qu'**un électrolyte aqueux basique contenant au moins un sel conducteur, choisi parmi les sels d'ammonium et de phosphonium bisquaternaires et multiquaternaires, est utilisé.

2. Procédé selon la revendication 1, **caractérisé en ce que** les esters α,β-insaturés sont utilisés avec les composés de carbonyle en un rapport essentiellement équimolaire.

3. Procédé selon la revendication 1 ou 2, **caractérisé en ce que** les réactifs esters α,β-insaturés et composés de carbonyle, ainsi que les produits du couplage croisé réducteur de ces réactifs se présentent sous la forme d'une émulsion dans l'électrolyte aqueux.

4. Procédé selon les revendications 1 à 3, **caractérisé en ce que** le sel conducteur est un sel d'ammonium ou de phosphonium bisquaternaire.

5. Procédé selon l'une quelconque des revendications 1 à 4, **caractérisé en ce que** le matériau de la cathode est choisi dans le groupe constitué par le plomb, les alliages de plomb, le cadmium, l'acier et le carbone vitreux.

6. Procédé selon l'une quelconque des revendications 1 à 5, **caractérisé en ce que** le matériau de l'anode est choisi dans le groupe constitué par l'acier au carbone, le carbone vitreux, l'acier, le mercure, le cadmium, le platine, le fer, le nickel, la magnétite, le plomb, les alliages de plomb ou le dioxyde de plomb.

7. Procédé selon l'une quelconque des revendications 1 à 6, **caractérisé en ce que** l'électrolyte aqueux contient au moins un tampon ayant une plage de tamponnage à un pH de 7 à 11.

8. Procédé selon l'une quelconque des revendications 1 à 7, **caractérisé en ce que** l'électrolyte aqueux contient en outre un ou plusieurs inhibiteurs de corrosion anodiques.

9. Procédé selon l'une quelconque des revendications 1 à 8, **caractérisé en ce que** l'électrolyte aqueux contient en outre un ou plusieurs complexants.

10. Procédé selon l'une quelconque des revendications 1 à 9, **caractérisé en ce que** le rapport molaire entre l'ester α,β-insaturé utilisé et le composé de carbonyle utilisé est compris entre 0,25 et 4.

11. Procédé selon l'une quelconque des revendications 1 à 10, **caractérisé en ce que** le composé de carbonyle est un aldéhyde.

12. Procédé selon l'une quelconque des revendications 1 à 11, **caractérisé en ce que** l'ester d'acide γ-hydroxycarboxylique formé est transestérifié lors d'une étape de procédé ultérieure en γ-lactone.

13. Procédé selon l'une quelconque des revendications 1 à 11, **caractérisé en ce que** la γ-lactone formée est transestérifiée lors d'une étape de procédé ultérieure en ester d'acide γ-hydroxycarboxylique.

14. Procédé selon l'une quelconque des revendications 1 à 11, **caractérisé en ce que** la γ-lactone formée et/ou l'ester d'acide γ-hydroxycarboxylique formé sont saponifiés lors d'une étape de procédé ultérieure en acide γ-hydroxycarboxylique.
